Europäisches Patentamt

European Patent Office

Office européen des brevets

(11) Veröffentlichungsnummer: **0 149 162 B1**

(12) # EUROPÄISCHE PATENTSCHRIFT

(45) Veröffentlichungstag der Patentschrift: 27.02.91

(21) Anmeldenummer: 84115535.1

(22) Anmeldetag: 15.12.84

(51) Int. Cl.⁵: **A01N 63/02**, A01N 63/00, C12N 15/32, //(C12P1/04, C12R1:07)

(54) **Neue, gegen Coleoptera wirksame Varietät des Bacillus thuringiensis sowie ein insektizid wirkendes, hieraus erhältliches Präparat bzw. Toxin sowie deren Verwendung zur Bekämpfung von Coleoptera.**

(30) Priorität: 21.12.83 DE 3346138

(43) Veröffentlichungstag der Anmeldung:
24.07.85 Patentblatt 85/30

(45) Bekanntmachung des Hinweises auf die
Patenterteilung:
27.02.91 Patentblatt 91/09

(84) Benannte Vertragsstaaten:
AT BE CH DE FR GB IT LI LU NL SE

(56) Entgegenhaltungen:
EP-A- 0 063 949
EP-A- 0 093 062

SOVIET INVENTIONS ILLUSTRATED, Section
CH: Chemical, Woche A05, 14. März 1978,
Referenz Nr. 9781, Derwent Publications,
London, GB and SU-A-548 625 (MORDOV
OGAREV UNIV) 09-01-1976

(73) Patentinhaber: BOEHRINGER MANNHEIM GMBH
Sandhofer Strasse 116
D-6800 Mannheim 31(DE)

(72) Erfinder: Krieg, Aloisius, Dr. rer. nat.
Schwarzer Weg 4
D-6100 Darmstadt(DE)
Erfinder: Huger, Alois, Dr. rer. nat.
Gersprenzweg 4
D-6100 Darmstadt-Eberstadt(DE)
Erfinder: Schnetter, Wolfgang, Dr.
Schubertstrasse 53
D-6901 Bammental(DE)

BIOLOGICAL ABSTRACTS, Band 77, Nr. 8, 1984, Seite 6409, Biosciences Information Service, Philadelphia, US; Referenz Nr. 58279; G. CANTWELL et al.: "Activity of the beta-exotoxin of Bacillus thuringiensis var. thuringiensis against the Colorado potato beetle [Leptinotarsa decemlineata] (Coleoptera: Chrysomelidae) and bacterial mutagenic response as determined by the Ames Test" and ENVIRON ENTOMOL 12(5) 1424-1427, 1983

ZEITSCHRIFT FÜR ANGEWANDTE ENTOMO-LOGIE, Band 96, 1983, Seiten 500-508, Hamburg, DE; A. KRIEG et al.: "Bacillus thuringiensis var. tenebrionis: ein neuer, gegenüber Larven von Coleopteren wirksamer Pathotyp"

# EP 0 149 162 B1

## Beschreibung

Neben den chemischen Insektiziden sind seit mehreren Jahren bakterielle Insektizide Gegenstand intensiver Forschungsarbeiten. Erstmals wurde 1915 der Bacillus thuringiensis (Bt) als Erreger der Schlaffsucht von Mehlmottenlarven beschrieben. Bis in die 70er Jahre gehörten alle Isolate von Bt einem Pathotyp an, der nur gegenüber Larven von Lepidopteren wirksam war. Dieser Pathotyp wird als Typ A bezeichnet. Zu ihm gehören außer der var. thuringiensis die ebenfalls praktisch benutzten Varietäten kurstaki und galleriae.

1977 isolierten Goldberg und Margalit aus einer von Mückenbrutplätzen in der Negev-Wüste stammenden Probe einen Stamm, der sich gegenüber Larven verschiedener Nematoceren (Diptera) als pathogen erwies und der später als Bacillus thuringiensis var. israelensis (Bti) klassifiziert wurde. Dieser Pathotyp wird als Typ B bezeichnet. Auch diese Varietät wird inzwischen wirtschaftlich genutzt.

Der Vorteil der bakteriellen Insektizide liegt darin, daß sie sehr selektiv auf bestimmte Schädlingsarten wirken.

So wird beispielsweise in dem US-Patent 4.166.112 ein Insektizid beansprucht, das die von Goldberg gefundene Varietät Bacillus thuringiensis var. israelensis als aktiven Bestandteil enthält. Hierin wird sehr deutlich gezeigt, wie selektiv dieser Bakterienstamm gegen Mückenlarven pathogen wirkt, während andere Organismen nicht beeinflußt werden.

Es besteht weiterhin Bedarf an bakteriellen Insektiziden gegen andere Schädlingsgruppen.

Gegenstand der Erfindung ist eine neue Varietät des Bacillus thuringiensis, die als Bacillus thuringiensis var. tenebrionis (Btt) bezeichnet wird. Es konnte gezeigt werden, daß dieser Stamm eine insektizide Wirkung, und zwar auf Käferarten (Coleoptera), insbesondere gegen Chrysomeliden, wie z. B. den Blauen Erlenblattkäfer (Agelastica alni) sowie den Kartoffelkäfer (Leptinotarsa decemlineata) besitzt. Es handelt sich als um einen neuen Pathotyp, der als Pathotyp C bezeichnet wird (siehe beispielsweise Zeitschrift für angewandte Entomologie, Band 96, Seiten 500-508 (1983) ). Ein weiterer Gegenstand der Erfindung ist ein bakterielles Insektizid, das als aktiven Wirkstoff Bacillus thuringiensis von Pathotyp C, insbesondere, Bacillus thuringiensis var. tenebrionis, oder ein hieraus erhältliches, insektizid wirkendes Präparat bzw. Toxin enthält.

Die neue Varietät Btt wurde isoliert aus kranken Entwicklungsstadien des Gemeinen Mehlkäfers (Tenebrio molitor L., Coleoptera: Tenebrionidae). Die Haltung der Reinkultur erfolgt bei 25-30°C auf Nähragar in Schrägröhrchen. Trockene Sporen wurden zur Typ-Konservierung eingefroren. Die Determination erfolgte aufgrund morphologischer und biochemischer Merkmale.

Das isolierte Bakterium produziert auf Nähragar große rauhe, weißliche Kolonien, deren Zellen nach 18 bis 24 Stunden sporulieren. Die vegetative Zelle ist begeißelt, stäbchenförmig und mißt etwa 1,0 x 4 - 8 $\mu$m. Sie reagiert grampositiv. Die Sporenmutterzelle enthält neben der subterminal gelegenen ellipsoidalen Spore (0,8 x 1,4 - 1,6 $\mu$m) einen parasporalen Kristall von flacher, plättchenartiger Form und mit dem Umriß eines Parallelogramms, Rhombus oder Quadrats (ca. 0,8 - 1,5 $\mu$m Kantenlänge).

In der vegetativen Phase erfolgt das Wachstum des Bacillus fakultativ auch anaerob. Die Sporulation ist dagegen strikt aerob. Das Temperaturoptimum für das Wachstum der vegetativen Zellen liegt bei 25-30°C. Autoklavieren 15 Minuten bei 121°C (entsprechend 1 bar Überdruck) inaktiviert die Sporen und auch das Kristalltoxin vollständig. Durch UV-Bestrahlung bei 254 nm werden die Sporen bei entsprechender Dosis inaktiviert, nicht aber das Kristalltoxin.

Die biochemischen Leistungen der vegetativen Zellen sind folgende. Aus abbaufähigen Kohlenhydraten (wie z. B. Glucose) wird Gärungssäure und Acetylmethylcarbinol gebildet, aber kein Gas. Außer Glucose werden unter Säuerung Mannose und Saccharose abgebaut, dagegen nicht Lactose, Cellobiose, Salicin, Arabinose, Xylose oder Mannit. Aesculin wird hydrolisiert, desgleichen Stärke. Es können folgende Enzyme nachgewiesen werden: Katalase, Nitratreduktase und Proteinase. Es konnten nicht nachgewiesen werden: Lysin-Decarboxylase, Phenylalanin-Desaminase, Urease und Lecithinase.

Für Produktionszwecke im Labormaßstab wurde ein Brutschüttler eingesetzt. In Erlenmeyerkolben von 250 ml wurden 50 ml Medium fermentiert. Das Medium hatte folgende Zusammensetzung: Hefeextrakt 0,5 %, Trypton 0,5 %, Glucose 0,1 %, $KH_2PO_4$ 0,08 % in Leitungswasser pH 7,0. Als Bebrütungstemperatur wurde 25 - 30°C gewählt. Im Anschluß an die Sporulation der Kultur, die mikroskopisch kontrolliert werden kann, wurde die Biomasse abzentrifugiert und mit Wasser oder Puffer ausgewaschen.

Die Wirkung von Btt auf Coleopteren-Larven folgt dem Schema, das bereits vom Pathotyp A bekannt ist: Es tritt Fraßstopp innerhalb weniger Stunden und Mortalität nach einigen Tagen in Abhängigkeit von der Dosis ein. Die toxische Wirkung beruht darauf, daß das Epithel des Mitteldarmes durch das im Darmsaft gelöste Kristalltoxin nachhaltig geschädigt wird. Die geschädigten Darmzellen lösen sich voneinander und von der Basalmembran und driften in das Darmlumen. Infolge des Zusammenbruchs der Darmschranke

3

können Bakterienzellen in das Hämocoel eindringen und dort den Prozeß der Desintegration durch eine tödliche Septikämie vollenden.

Die neue Varietät Btt, Pathotyp C, deren Prototyp aus einer toten Mehlkäferpuppe isoliert werden konnte, wurde bei der Deutschen Sammlung für Mikroorganismen unter der Nr. DSM 2803 hinterlegt. Nach der serologischen Untersuchung gehört die var. tenebrionis, Stamm DSM 2803, zum Serotyp H 8a, 8b.

Die neue Varietät Btt, Pathotyp C, zeigt eine selektive insektizide Wirkung auf Coleoptera, beispielsweise auf den Blauen Erlenblattkäfer oder den Kartoffelkäfer. Die Wirkung ist bei Larven wesentlich stärker als bei Imagines. Keine Wirkung konnte beobachtet werden gegen Lepidoptera, beispielsweise gegenüber der Mehlmotte (Ephestia Kühniella Zell.), Kohlmotte (Plutella xylostella L.) und auch nicht gegenüber Diptera, beispielsweise der Gelbfiebermücke (Aedes aegypti L.). In der folgenden Tabelle sind die Ergebnisse der durchgeführten Biotests zusammengestellt.

Toxische Wirkung von Btt auf verschiedene Coleoptera, Lepidoptera bzw. Diptera

| Untersuchte Art | Eingesetzte Dosis [Sporenäquivalente] | tox. Wirkung |
|---|---|---|
| Blauer Erlenblatt-käfer | $10^6/cm^2$ | + |
| Kartoffelkäfer | $10^6/cm^2$ | + |
| Mehlmotte | $2,5 \times 10^8/cm^3$ | - |
| Kohlmotte | $4 \times 10^7/cm^2$ | - |
| Gelbfiebermücke | $5 \times 10^6/cm^3$ | - |

Das eigentliche insektizide Wirkprinzip bei Bacillus thuringiensis ist das Kristalltoxin. Die Kristallmasse kann isoliert werden, indem man bei den sporulierten Kulturen nach der Lyse der Sporangien die Kristalle von den Zellresten und den Sporen trennt und reinigt, beispielsweise über Dichtegradienten.

Bei der Molekulargewichtsbestimmung mit Hilfe der SDS-Polyacrylamidgelelektrophorese nach der Methode von Laemmli [Nature 227 , 680 (1970)] werden zwei Hauptbanden bei ca. 65 000 D und ca. 70 000 D gefunden. Kleinere Banden von geringerer Intensität liegen im Bereich von 20.000 bis 40.000 D.

Mit Hilfe des erfindungsgemäßen Toxins können Antikörper gewonnen werden, die im Immunodiffusionstest nach Ouchterlony keine Kreuzreaktion mit anderen Toxinen des Pathotyps A oder B zeigen.

Das Toxin wird von einem Gen codiert, das in einem Plasmid enthalten ist. Das Toxin wird auch in asporogenen Mutanten produziert, die durch Behandlung mit Ethylmethan-sulfonat erzeugt werden. Die Fähigkeit zur Toxinproduktion kann durch Plasmid- oder Gentransfer auf andere Bacillus thuringiensis Varietäten oder andere Bakteriensysteme übertragen werden. Die insecticide Wirkung beruht auf dem Vorhandensein des plasmidcodierten Toxins.

Zur Anwendung als Insektizid wird Bacillus thuringiensis var. tenebrionis oder ein hieraus erhältliches, insektizid wirkendes Präparat bzw. Toxin in an sich bekannter Weise mit üblichen Additiven (Trägerstoffe, Haftmittel, Netzmittel etc.) versetzt und in eine geeignete Anwendungsform übergeführt. Das so formulierte Insektizid kann in Form eines Spritzpulvers, einer Suspension, als Granulat o. ä. eingesetzt werden. Bevorzugt wird eine gereinigte, sporulierte Kultur in Form einer Suspension unter Zusatz eines Netzmittels, beispielsweise 0,1 % Citowet[R] (BASF), eingesetzt. Pro Hektar zu behandelndes Feld werden $10^{12}$ bis $10^{15}$, vorzugsweise $10^{13}$ bis $10^{14}$ Sporen und eine äquivalente Anzahl von toxischen Kristallen versprüht. Auf diese Weise gelingt es zum Beispiel ein Kartoffelfeld 2 bis 4 Wochen lang vor dem Kartoffelkäfer zu schützen. Zum Schutze während der gesamten Wachstumsphase sind weitere Behandlungen in dem genannten zeitlichen Abstand erforderlich. Eine Wirkung gegen Nicht- Zielorganismen, wie beispielsweise gegen Entomophagen und Honigbienen, war nicht zu beobachten.

# EP 0 149 162 B1

## Ansprüche

1. Bacillus thuringiensis, dadurch gekennzeichnet, daß er ein Toxin mit einem Molekulargewicht von ca. 65000 - 70000 Dalton produziert, das selektiv gegen Coleoptera wirksam ist und das Bakterium nicht den Pathotypen A oder B angehört.

2. Bacillus thuringiensis var. tenebrionis, dadurch gekennzeichnet, daß er ein Toxin mit einem Molekulargewicht von ca. 65000 - 70000 Dalton produziert, das selektiv gegen Coleoptera wirksam ist und das Bakterium nicht den Pathotypen A oder B angehört.

3. Bacillus thuringiensis var. tenebrionis DSM 2803.

4. Toxin, das selektiv gegen Coleoptera wirksam ist, ein Molekulargewicht von ca. 65000 - 70000 Dalton aufweist und aus einem Bacillus thuringiensis, gemäß Anspruch 1 erhältlich ist.

5. Verfahren zur Gewinnung eines Toxins gemäß Anspruch 4, dadurch gekennzeichnet, daß man bei Kulturen eines Bacillus thuringiensis gemäß Anspruch 1 nach der Lyse der Sporangien die Kristalle von den Sporen und Zellresten trennt und die Kristallmasse reinigt.

6. Plasmid, das exprimierbare DNA enthält, die für ein Toxin gemäß Anspruch 4 codiert.

7. DNA mit einer Nukleotidsequenz, die für ein Toxin codiert, das selektiv gegen Coleoptera wirksam ist, ein Molekulargewicht von ca. 65000 - 70000 Dalton aufweist und aus einem Bacillus gemäß einem der Ansprüche 1 bis 3 erhältlich ist.

8. Bacterium, das ein Plasmid gemäß Anspruch 6 oder DNA gemäß Anspruch 7 enthält.

9. Verwendung eines Bacillus thuringiensis gemäß Anspruch 1 oder eines hieraus erhältlichen Toxins zur Bekämpfung von Coleoptera.

10. Insektizid, dadurch gekennzeichnet, daß es als aktiven Wirkstoff einen Bacillus thuringiensis gemäß Anspruch 1 oder ein hieraus erhältliches Toxin enthält.

11. Insektizid gemäß Anspruch 10, dadurch gekennzeichnet, daß es Bacillus thuringiensis var. tenebrionis DSM 2803 oder ein hieraus erhältliches Toxin enthält.

## Claims

1. Bacillus thuringiensis, characterised in that it produces a toxin with a molecular weight of about 65000 - 70000 Dalton which is selectively effective against Coleoptera and the Bacterium does not belong to the pathotypes A or B.

2. Bacillus thuringiensis var. tenebrionis, characterised in that it produces a toxin with a molecular weight of about 65000 - 70000 Dalton which is selectively effective against Coleoptera and the Bacterium does not belong to the pathotypes A or B.

3. Bacillus thuringiensis var. tenebrionis DSM 2803.

4. Toxin which is selectively effective against Coleoptera, has a molecular weight of about 65000 - 70000 Dalton and is obtainable from a Bacillus thuringiensis according to claim 1.

5. Process for the obtaining of a toxin according to claim 4, characterised in that, in the case of cultures of a Bacillus thuringiensis according to claim 1, after lysis of the sporangia, one separates the crystals from the spores and cell residues and purifies the crystal mass.

6. Plasmid which contains expressable DNA which codes for a toxin according to claim 4.

7. DNA with a nucleotide sequence which codes for a toxin which is selectively effective against Coleoptera, has a molecular weight of about 65000 - 70000 and is obtainable from a Bacillus according to one of claims 1 to 3.

8. Bacterium which contains a plasmid according to claim 6 or DNA according to claim 7.

9. Use of a Bacillus thuringiensis according to claim 1 or of a toxin obtainable herefrom for the combating of Coleoptera.

10. Insecticide, characterised in that it contains, as active material, a Bacillus thuringiensis according to claim 1 or a toxin obtainable herefrom.

11. Insecticide according to claim 10, characterised in that it contains Bacillus thuringiensis var. tenebrionis DSM 2803 or a toxin obtainable herefrom.

**Revendications**

1. Bacillus thuringiensis, caractérisé en ce qu'il produit une toxine d'un poids moléculaire d'environ 65 000 à 70 000 daltons, qui est sélectivement active contre les coléoptères, et la bactérie n'appartient pas au pathotype A ou B.

2. Bacillus thuringiensis var. tenebrionis, caractérisé en ce qu'il produit une toxine d'un poids moléculaire d'environ 65 000 à 70 000 daltons, qui est sélectivement active contre les coléoptères, et la bactérie n'appartient pas au pathotype A ou B.

3. Bacillus thuringiensis var. tenebrionis DSM 2803.

4. Toxine, qui est sélectivement active contre les coléoptères, a un poids moléculaire d'environ 65 000 à 70 000 daltons et peut être obtenue à partir d'un Bacillus thuringiensis suivant la revendication 1.

5. Procédé pour la préparation d'une toxine suivant la revendication 4, caractérisé en ce qu'on sépare les cristaux des spores et débris cellulaires après la lyse des sporanges dans des cultures d'un Bacillus thuringiensis suivant la revendication 1 et on purifie la masse cristalline.

6. Plasmide, qui contient de l'ADN exprimable qui code pour une toxine suivant la revendication 4.

7. ADN, comprenant une séquence de nucléotides qui code pour une toxine qui est sélectivement active contre les coléoptères, a un poids moléculaire d'environ 65 000 à 70 000 daltons et peut être obtenue à partir d'un bacille suivant l'une quelconque des revendications 1 à 3.

8. Bactérie, qui contient un plasmide suivant la revendication 6 ou de l'ADN suivant la revendication 7.

9. Utilisation d'un Bacillus thuringiensis suivant la revendication 1 ou d'une toxine qui peut en être obtenue pour la lutte contre les coléoptères.

10. Insecticide, caractérisé en ce qu'il contient comme principe actif un Bacillus thuringiensis suivant la revendication 1 ou une toxine qui peut en être obtenue.

11. Insecticide suivant la revendication 10, caractérisé en ce qu'il contient Bacillus thuringiensis var. tenebrionis DSM 2803 ou une toxine qui peut en être obtenue.